(19) **Europäisches Patentamt**
**European Patent Office**
**Office européen des brevets**

(11) **EP 2 271 901 B1**

(12) **EUROPEAN PATENT SPECIFICATION**

(45) Date of publication and mention of the grant of the patent:
**30.11.2016 Bulletin 2016/48**

(21) Application number: **09721636.0**

(22) Date of filing: **19.03.2009**

(51) Int Cl.:
*G01J 3/40* (2006.01)    *G02F 1/01* (2006.01)
*G01N 21/25* (2006.01)    *A61B 6/02* (2006.01)
*A61B 5/00* (2006.01)    *G01J 3/02* (2006.01)
*G01J 3/28* (2006.01)    *G01N 21/31* (2006.01)
*A61B 5/1455* (2006.01)

(86) International application number:
**PCT/US2009/037706**

(87) International publication number:
**WO 2009/117603 (24.09.2009 Gazette 2009/39)**

(54) **MINIATURIZED MULTI-SPECTRAL IMAGER FOR REAL-TIME TISSUE OXYGENATION MEASUREMENT**

MINIATURISIERTER MULTISPEKTRALBILDGEBER FÜR ECHTZEIT-GEWEBE-OXIGENATIONS-MESSUNGEN

IMAGEUR À SPECTRES MULTIPLES MINIATURISÉ POUR UNE MESURE EN TEMPS RÉEL DE L'OXYGÉNATION D'UN TISSU

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **19.03.2008 US 37780 P**

(43) Date of publication of application:
**12.01.2011 Bulletin 2011/02**

(73) Proprietor: **HyperMed Imaging, Inc.**
**Memphis TN 38125 (US)**

(72) Inventors:
• **LIFSITZ, Rick**
  **Wellesley**
  **MA 02481 (US)**
• **GUSYATIN, Oleg**
  **Weston**
  **MA 02493 (US)**
• **STONEHAM, Marie**
  **Weston**
  **MA 02493 (US)**

• **SHUBENTSOV, Llya**
  **Weston**
  **MA 02493 (US)**
• **JIANG, Chunsheng**
  **Weston**
  **MA 02493 (US)**

(74) Representative: **Grund, Martin**
**Grund Intellectual Property Group**
**Patentanwalt und Solicitor PartG mbB**
**Nikolaistrasse 15**
**80802 München (DE)**

(56) References cited:
**US-A- 4 947 850        US-A- 5 438 989**
**US-A1- 2007 024 946      US-A1- 2007 268 485**
**US-A1- 2008 188 758      US-B2- 6 728 561**

• **DAISUKE NAKAO ET AL.: 'Real time multi spectral image processing for mapping pigmentation in human skin' MED IMAGING TECHNOL vol. 20, no. 2, 2002, pages 123 - 133, XP008142850**

**Description**

**Background**

**1. Field of the Invention**

The invention is directed to multi-spectral imaging, Specifically, the invention is directed to a portable multi-spectral imager.

**2. Background of the Invention**

Spectroscopy, whether it is visible, near infrared, infrared or Raman, is an enormously powerful tool for the analysis of biomedical samples. The medical community, however, has a definite preference for imaging methods, as exemplified by methods such as MRI and CT scanning as well as standard X-ray photography and ultrasound imaging. This is entirely understandable as many factors need to be taken into account for a physician to make a clinical diagnosis. Imaging methods potentially can provide far more information to a physician than their non-imaging counterparts. With this medical reality in mind, there has been considerable effort put into combining the power and versatility of imaging method with the specificity of spectroscopic methods.

Near-infrared (near-IR) spectroscopy and spectroscopic imaging can measure the balance between oxygen delivery and tissue oxygen utilization by monitoring the hemoglobin oxygen saturation in tissues (Sowa, M. G. et al., 1998, Proc. SPIE 3252, pp. 199-207; Sowa, G. W. et al., 1999, Journal of Surgical Research, 86:62-29; Sow, G. W. et al.,1999, Journal of Biomedical Optics, 4:474-481; Mansfield, J. R., et al., 2000, International Society of Optical Engineers, 3920:99-197). For in-vivo human studies, the forearm or leg has been the investigational site for many of the noninvasive near-IR studies. Non-imaging near-IR applications have examined the local response of tissue to manipulations of blood flow (De-Blasi, R. A. et al., 1992, Adv. Exp. Med. Biol, 317:771-777). Clinically, there are situations where the regional variations in oxygenation saturation are of interest (Stranc, M. F. et al, 1998, British Journal of Plastic Surgery, 51:210-218). Near-IR imaging offers a means of accessing the spatial heterogeneity of the hemoglobin oxygenation saturation response to tissue perfusion. (Mansfield, J. R. et al., 1997, Analytical Chemistry, 69:3370-3374; Mansfield, J. R., et al., 1997, Computerized Medical Imaging and Graphics, 21:299-308; Salzer, R., et al., 2000, Fresenius Journal of Analytical Chemistry, 366:712-726; Shaw, R. A., et al., 2000, Journal of Molecular Structure (Theochem), 500:129-138; Shaw, R. A., et al., 2000, Journal of Inorganic Biochemistry, 79:285-293; Mansfield, J. R., et al., 1999, Proc. SPIE Int. Soc. Opt. Eng., 3597:222-233; Mansfield, J. R., et al., 1999, Applied Spectroscopy, 53:1323-1330; McIntosh, L. M., et al., 1999, Biospectroscopy, 5:265-275; Mansfield, R., et al., Vibrational Spectroscopy, 19:33-45; Payette, J. R., et al., 1999, American Clinical Laboratory, 18:4-6; Mansfield, J. R., ct al., 1998, IEEE Transactions on Medical Imaging, 6:1011-1018.

Non-invasive monitoring of hemoglobin oxygenation exploits the differential absorption of $HbO_2$ and Hb, along with the fact that near-IR radiation can penetrate relatively deeply into tissues. Pulse oximetry routinely supplies a noninvasive measure of arterial hemoglobin oxygenation based on the differential red-visible and near infrared absorption of Hb and HbO.sub.2. Visible/near-IR multispectral imaging permits the regional variations in tissue perfusion to be mapped on macro and micro scale. Unlike infrared thermography, hyperspectral imaging alone does not map the thermal emission of the tissues. Instead, this imaging method relies on the differential absorption of light by a chromophore, such as, Hb and HbO.sub.2, resulting in differences in the wavelength dependence of the tissue reflectance depending on the hemoglobin oxygen saturation of the tissue. (Sowa, M. G., et al., 1997, Applied Spectroscopy, 51:143-152; Leventon, M., 2000, MIT Ph.D. Thesis).

Spectroscopic imaging methodologies and data are becoming increasingly common in analytical laboratories, whether it be magnetic resonance (MRI), mid-IR, Roman, fluorescence and optical microscopy, or near-IR/visible-based imaging. However, the volume of information contained in spectroscopic images can make standard data processing techniques cumbersome. Furthermore, there are few techniques that can demarcate which regions of a spectroscopic image contain similar spectra without a priori knowledge of either the spectral data or the sample's composition. The objective of analyzing spectroscopic images is not only to determine what the spectrum is at any particular pixel in the sample, but also to determine which regions of the sample contain similar spectra; i.e., what regions of the sample contain chemically related compounds. Multivariate analysis methodologies can be used to determine both the spectral and spatial characteristics of a sample within a spectroscopic imaging data set. These techniques can also be used to analyze variations in the temporal shape of a time series of images either derived for extracted from a time series of spectroscopic images. US2007/024946 describes a portable multi-spectral imaging device comprising an image acquisition device configured to acquire a multi-spectral image from a subject, a filtering device to filter light received by the image acquisition device, and a diagnostic processor. US2008/188758 relates to a passive skin detection system for determining the presence of skin in a captured image. However, neither of these disclosures provides a device that safeguards the acquired data through a continuous monitoring of data quality with respect to the achieved signal to noise ratio, or real-time motion tracking to prevent data corruption.

There are few techniques that can demarcate which regions of a sample contain similar substances without a priori knowledge of the sample's composition. Spectroscopic imaging provides the specificity of spectroscopy while at the same time relaying spatial information by providing images of the sample that convey some chemical meaning. Usually the objective in analyzing heterogeneous systems is to identify not only the components present in the system, but their spatial distribution. The true power of this technique relative to traditional imaging methods lies in its inherent multivariate nature. Spatial relationships among many parameters can be assessed simultaneously. Thus, the chemical lieterogeneity or regional similarity within a sample is captured in a high dimensional representation which can be projected onto a number of meaningful low dimensional easily interpretable representations which typically comprise a set of composite images each having a specific meaning.

While it is now clear that both spectroscopy and spectroscopic imaging can play roles in providing medially relevant information, the raw spectral or imaging measurement seldom reveals directly the property of clinical interest. For example using spectroscopy, one cannot easily determine whether the tissue is cancerous, or determine blood glucose concentrations and the adequacy of tissue perfusion. Instead, pattern recognition algorithms, clustering methods, regression and other theoretical methods provide the means to distill diagnostic information from the original analytical measurements.

There are however various methods for the collection of spectroscopic images. In all such cases, the result of a spectroscopic imaging experiment is something termed a spectral image cube, spectroscopic imaging data cube or just hypercube. This is a three dimensional array of data, consisting of two spatial dimensions (the imaging component), and one spectral dimension. It can be thought of as an array of spatially resolved individual spectra, with every pixel in the first image consisting of an entire spectrum, or as a series of spectrally resolved images. In either representation, the 3D data cube can be treated as a single entity containing enormous amounts of spatial and spectral information about the sample from which it was acquired.

As an extension of the three dimensional array acquired in a spectroscopic imaging experiment, one can collect data cubes as a function of additional parameters such as time, temperature or pH. Numerous algorithms can be used to analyze these multi-dimensional data sets so that chemical and spectral variations can be studied as additional parameters. However, taken together, they can allow one to more fully understand the variations in the data. This can be done in a gated or sequential fashion.

Multi-modal image fusion, or image registration, is an important problem frequently addressed in medical image analysis. Registration is the process of aligning data that arise from different sources into one consistent coordinate frame. For example, various tissues appear more clearly in different types of imaging methods. Soft tissue, for example, is imaged well in MR scans, while bone is more easily discernible in CT scans. Blood vessels are often highlighted better in an MR angiogram than in a standard MR scan. Multiple scans of the same patient will generally be unregistered when acquired, as the patient may be in different positions in each scanner, and each scanner has its own coordinate system. In order to fuse the information from all scans into one coherent frame, the scans must be registered. The very reason why multiple scans are useful is what makes the registration process difficult. As each modality images tissue differently and has its own artifacts and noise characteristics, accurately modeling the intensity relationship between the scans, and subsequently aligning them, is difficult.

The registration of two images consists of finding the transformation that best maps one image into the other. If $I_1$ and $I_2$ are two images of the same tissue and T is the correct transformation, then the voxel $I_1(x)$ corresponds to the same position in the sample as the voxel $I_2(T(x))$. In the simplest case, T is a rigid transformation consisting of three degrees of freedom of rotation and three degrees of freedom of translation. The need for rigid registration arises primarily from the patient being in different positions in the scanning devices used to image the anatomy. The information from all the images is best used when presented in one unified coordinate system. Without such image fusion, the clinician must mentally relate the information from the disparate coordinate frames.

One method of aligning the two images is to define an intermediate, patient-centered coordinate system, instead of trying to directly register the images to one another. An example of a patient-centered reference frame is the use of fiducial markers attached to a patient throughout the various image acquisitions. The fiducial markers define a coordinate system specific to the patient, independent of the scanner or choice of imaging modality. If the markers remain fixed and can be accurately localized in all the images, then the volumes can be registered by computing the best alignment of the corresponding fiducial (Horn, B. K. P., 1987, Journal of the Optical Society of America A, 4:629-642; Mandava, V. R., et al., Proc SPIE, 1992, 1652:271-282; Haralick, R. M., et al., 1993, Computer and Robot Vision). The main drawback of this method is that the markers must remain attached to the patient at the same positions throughout all image acquisitions. For applications such as change detection over months or years, this registration method is not suitable. Fiducial registration is typically used as ground-truth to evaluate the accuracy of other methods as careful placement and localization of the markers can provide very accurate alignment (West, J. et al., 1996, Proc SPIE, Newport Beach, Calif.).

When fiducial markers are not available to define the patient coordinate frame, corresponding anatomical feature points can be extracted from the images and used to compute the best alignment (Maintz, J. B. Antione, et al., 1995 Computer Vision, Virtual Reality and Robotics in Medicine, pp. 219-228; Maguire, Jr., G., et al., 1991, IEEE Computer Graphics Applications, 11:20-29). This approach depends greatly on the ability to automatically and accurately extract reliable image features. In general, methods of feature extraction such as intensity thresholding or edge detection do not work well on medical scans, due to non-linear gain fields and highly textured structures. Even manual identification of corresponding 3D anatomical points can be uireliable. Without the ability to accurately localize corresponding features in the images, alignment in this manner is difficult.

Instead of localizing feature points in the images, richer structures such as object surfaces can be extracted and used as a basis of registration. A common method of registering MR and CT of the head involves extracting the skin (or skull) surfaces from both images, and aligning the 3D head models (Jiang, H., et al., 1992 Proc. SPIE, 1808:196-213; Lemoine, D. et al., 1994, Proc. SPIE, 2164:46-56). For PET/MR registration, the brain surface is typically used since the skull is not clearly visible in PET (Pelizzari, C., et al., J Comput Assist. Tumour., 1989, 13:20-26). The 3D models are then rigidly registered using surface-based registration techniques (Ettinger, G., 1997, MIT Ph.D Thesis). The success of such methods relies on the structures being accurately and consistently segmented across modalities and the surfaces having rich enough structure to be unambiguously registered.

Voxel-based approaches to registration do not extract any features from the images, but use the intensities themselves to register the two images. Such approaches model the relationships between intensities of the two images when they are registered, and then search through the transformation space to find an alignment that best agrees with the model. Various intensity models are discussed, including correlation, mutual information, and joint intensity priors.

Correlation is a measure commonly used to compare two images or regions of images for computer vision problems such as alignment or matching. Given the intensity values of two image patches stacked in the vectors u and v, the normalized correlation measure is the dot product of unit vectors in the directions of u and v:

$$(u \cdot v)/(\|u\|\|v\|)$$

An advantage of correlation-based methods is that they can be computed quite efficiently using convolution operators. Correlation is applicable when one expects a linear relationship between the intensities in the two images. In computer vision problems, normalized correlation provides some amount of robustness to lighting variation over a measure such as sum of square differences (SSD), $\|u-v\|^2$. The primary reason for acquiring more than one medical scan of a patient stems from the fact that each scan provides different information to the clinician. Therefore, two images that have a simple linear intensity relationship may be straightforward to register, but do not provide any additional information than one scan by itself. On the other hand, if the images are completely independent (e.g. no intensity relationship exists between them), then they cannot be registered using voxel-based methods. In general, there is some dependence between images of different modalities and each modality does provide additional information.

One simplified model of the medical imaging process is that an internal image is a rendering function R of underlying tissue properties, P(x), over positions x. An image of modality A could be represented as a function $R_A(P)$ and a registered image of modality B of the same patient would be another function, say $R_B(P)$. Suppose a function F(x) could be computed relating the two rendering functions such that the following is true (with the possible addition of some Gaussian noise, N):

$$F(R_B(P)) = R_A(P) + N$$

The function F would predict the intensity at a point in Image A given the intensity at the corresponding point in Image B. Such a function could be used to align a pair of images that are initially in different coordinate systems using SSD:

$$T^* = \mathrm{argmin}_T \Sigma_x (F(R_B(P(X))) - R_A(P(x)))^2$$

where T is the transformation between the two sets of image coordinates. Van den Elsen et al. compute such a mapping that makes a CT image appear more like an MR, and then register the images using correlation (van den Elsen, P., et al., 1994, "Visualization in Biomedical Computing," 1994 Proc SPIE, 2359:227-237). In general, explicitly computing the function F that relates two imaging modalities is difficult and under-constrained.

Maximization of mutual information (MI) is a general approach applicable to a wide range of multi-modality registration applications (Bell, A. J., et al., 1995 Advances in Neural Information Processing 7; Collignon, D., et al., 1995, First Conf. on Computer Vision, Virtual Reality and Robotics in Medicine Springer; Maes, F. et al, 1996, Mathematical Methods in Biomedical Image Analysis; Wells, W. M., et al., 1996, Medical Image Analysis, 1(1):35-51). One of the strengths of using mutual information is that MI does not use any prior information about the relationship between joint intensity distributions. While mutual information does not explicitly model the function F that relates the two imaging modalities, it assumes that when the images are aligned, each image should explain the other better than when the images are not aligned.

Given two random variables U and V, mutual information is defined as (Bell, 1995):

$$MI(U,V)=H(U)+H(V)-H(U,V)$$

where H(U) and H(V) are the entropies of the two variables, and H(U,V) is the joint entropy. The entropy of a discrete random variable is defined as:

$$H(U)=-\Sigma P_u(u) \log P_u(u)$$

where P.sub.u (u) is the probability mass function associated with U. Similarly, the expression for joint entropy entropy operates over the joint PDF:

$$H(U,V)=-\Sigma\Sigma P_{u,v}(u,v) \log P_{u,v}(u,v)$$

When U and V are independent, H(U,V)=H(U)+H(V), which implies the mutual information is zero. When there is a one-to-one functional relationship between U and V, (i.e. they are completely dependent), the mutual information is maximized as:

$$MI(U,V)=H(U)=H(V)=H(U,V)$$

To operate on images over a transformation, we consider the two images, I.sub.1 (x) and I.sub.2 (x) to be random variables under a spatial parameterization, x. We seek to find the value of the transformation T that maximizes the mutual information (Wells, 1996):

$$T^*=\text{argmax}_T MI(I_1(x),I_2(T(x)))$$

$$T^*=\text{argmax}_T H(I_1(x))+H(I_2(T(x)))-H(I_1(x),I_2(T(x)))$$

The entropies of the two images encourage transformations that project I1 onto complex parts of I2. The third term, the (negative) joint entropy of $I_1$ and $I_2$, takes on large values when X explains Y well. Derivatives of the entropies with respect to the pose parameters can be calculated and used to perform stochastic gradient ascent (Wells, 1996). West et al. compare many multi-modal registration techniques and find mutual information to be one of the most accurate across all pairs of modalities (West, 1996).

Leventon et al. introduced an approach to multi-modal registration using statistical models derived from a training set of images (Leventon, M., et al., 1998, Medical Image Computing and Computer-assisted Intervention). The method involved building a prior model of the intensity relationship between the two scans being registered. The method requires a pair of registered training images of the same modalities as those to be registered in order to build the joint intensity model. To align a novel pair of images, the likelihood of the two images given a certain pose based on our model by sampling the intensities at corresponding points is computed. This current hypothesis can be improved by ascending the log likelihood function. In essence, one computes a probabilistic estimate of the function F (that relates the two imaging modalities) based on intensity co-occurrence. To align the novel images, the pose is found that maximizes the likelihood that those images arose from the same relation F.

Building a joint-intensity model does require having access to a registered pair of images of the same modality and approximately the same coverage as the novel pair to be registered. Mutual information approaches do not need to draw upon previously registered scans. However, when this information is available, the prior joint intensity model provides the registration algorithm with additional guidance which results in convergence on the correct alignment more quickly, more reliably and from more remote initial starting points.

## Summary of the Invention

The present invention, as embodied in the appended claims, overcomes the problems and disadvantages associated with current designs of existing hyperspectral imaging devices. In particular, the device provides for real-time measurements of patients oxygenation levels and other components which are specific to a disease condition. The present invention expands on the invention disclosed in U.S. Patent No. 6,640,130. Furthermore, it is an improvement over the existing device in that this is done in real time.

A portable multi-spectral imaging system is disclosed.

The system includes at least one image acquisition device for acquiring an image from a subject, a filtering device to filter the light received by the image acquisition device, a processor for processing the image acquired by the image acquisition device, and a display. There is software running on the processor that determines oxygenation values or other relevant components of the subject based on the processed image.

The oxygenation values may be based on at least one of oxyhemoglobin, deoxyhemoglobin and oxygen saturation levels or on other relevant components. The software may filter the image to reduce noise, correct at least one of the images to account for motion of the subject, eliminates extraneous objects from the acquired image, and/or compare the data of the acquired image to stored data.

The system may also include an illumination source and may communicate, through wireless or wired channels, with an analysis device. The analysis device may compare multiple images, store acquired images, and/or store acquired oxgynation values.

The invention is directed to a portable multi-spectral imaging device. The device includes at least one image acquisition device for acquiring a multi-spectral image from a subject, a filtering device to filter the light received by the image acquisition device, an acquisition subsystem configured to continuously monitor quality of acquired image data with respect to the signal-to-noise ratio achieved, and perform motion tracking in real-time such that acquired data is not corrupted during imaging an analogue front end module to convert the image to a digital image, a microprocessor to temporarily store at least one image and control the analogue front end module, and a communications module configured to send image data through a wireless connection for storage.

The image may be used to determine oxygenation values of the subject The oxygenation values may be based on at least one of oxy-hemoglobin, de-oxyhemoglobin and/or oxygen saturation levels.

The device may also include an illumination source that may produce filtered light. The device may have a maximum diameter of 5,08 cm (2 inches), and/or be handheld. The device may further include a power source.

## Description of the Drawings

The invention is described in greater detail by way of example only and with reference to the attached drawings, in which:

Figure 1 is a 3-D view of an embodiment of a multi-spectra imaging device.
Figure 2 is an exploded view of Figure 1.
Figure 3 is a schematic block diagram of the multi-spectral imaging device.
Figures 4a-4 show a second embodiment of a multi-spectra imaging device.

## Description of the Invention

As embodied and broadly described herein, the disclosures herein provide detailed embodiments of the invention..

A problem in the art capable of being solved by the embodiments of the present invention is producing a miniaturized medical multi-spectral imaging (MSI) sensor capable of providing real-time measurements of oxygen saturation $S_1O_2$ in skin, serving as an excellent indicator of oxygenation status in patients with multiple medical conditions, including but not limited to diabetes, wound care, vascular disease and pressure ulcers as well as providing an early warning for the onset of shock. It has been surprisingly discovered that a highly sensitive miniature sensor may be capable of precise early measurements of oxygen levels in the skin of patients at risk of diabetes, wound care, vascular disease, pressure ulcers or other disease states.. The device may be optimized to support field-deployable, portable operational scenarios for remote and extreme environments, both military and civilian. Furthermore, by reducing costs, the hand-held device may be used by patients at risk for developing diabetic foot ulcers with the aim of warning for early tissue breakdown, thereby preventing ulceration.

The miniature multi-spectral imaging device is likely to have superior qualities in comparison to other currently existing point measuring near-infrared spectroscopy (NIR) devices. This is due to the fact that the device predominantly measures $SO_2$ in the skin capillary bed. Therefore, skin thickness and fat layers have less affect on the returned signal whereas NIR based technologies need to actively correct for these factors.

The device's sensor has cross polarized illumination detection which is less sensitive to surface glare and superficial scattering. Furthermore, it generates a more advanced hemoglobin decomposition algorithm. Crosstalk between de-oxyhemoglobin and other background terms was noted in previous algorithms leading to high variability in the de-oxyhemoglobin signal and in the oxygen saturation value determined from it.

It is possible to correlate the spectrum of each pixel with the presence and concentration of various chemical species. This data can then be interpreted as a "gradient map" of these species in a surface. HSI for medical applications (MHSI) has been shown to accurately predict viability and survival of tissue deprived of adequate perfusion, and to differentiate diseased tissue (e.g. tumor) and ischemic tissue from normal tissue. MHSI analysis uses spatial and spectral characteristics obtained from the skin to develop indices for shock prediction based primarily on oxy- and deoxy-Hb signals including (1) average index (mean across image), (2) heterogeneity index (inter-quartile range), (3) mottling index (analysis of spatial features) and (4) temporal shift index (change in mottling pattern from one image to the next). Using a combination of these techniques, a hyperspectral index (HSI) as a simple numerical reading has been developed. HSI may serve as an early indicator of the many disease states such as vascular disease, diabetes, pressure ulcers and shock..

The handheld multi-spectral imaging system is designed for tissue optical imaging for non-contact evaluation of tissue oxygenation, over an extended area, without harmful radiation, and without the need of using any agent into the tissue. Due to its compact design with a fast image sensor, high-efficient illuminator and high-speed wavelength selection, it may be entirely portable and self-contained to acquire image data and provide hyperspectral information of tissue oxygenation to the user.

The system includes an imaging acquisition parameter dynamics module. This module is responsible for on-the-fly adjustment of image sensor parameters in order to ensure that SNR (Signal-to-Noise) requirements are met for a given target (i.e. skin type, surface type, etc.). In addition, accommodations are made to handle various lighting conditions present during acquisition phase. The module is responsible for choosing appropriate wavelengths and controlling high-speed wavelength selector. High-efficiency illumination may also be controlled and is adjusted in real-time based on surface reflectance characteristics used as feedback at a given wavelength of light.

It is important to enforce acquisition parameters such that a general imaging problem is constrained. A fiducial metrics module is responsible for locating fiducial marks and executing the device's spatial positioning/stabilization logic relative to surface to be imaged in real-time. Thus, optimizing illumination delivery as well as positioning repeatability.

In parallel with other tasks, an acquisition subsystem continuously monitors quality of the data with respect to achieved SNR. Since sought imaging modality is unconstrained relative to patient's motion (by design, to deliver robust usage model) motion tracking is also performed in real-time to ensure that data is not corrupted during imaging.

Overall, the acquisition platform is designed to deliver accurate real-time performance due to custom hardware (truly parallelized microprocessors, FPGAs)/software implementation (embedded) that incorporates observed feedback with a priori knowledge about imaging modality to produce unparalleled performance and data quality. The major image processing software is designed and developed to achieve adaptive filtering, fast and accurate image registration, fast and effective tissue/obstruction masking and high performance algorithm for tissue oxygenation values such as oxyhemoglobin, deoxyhemoglobin and oxygen saturation.

The software includes adaptive spatial filtering, which will take place in order to ensure that noise is minimized without compromising informative data. The software further includes spatial registration, which is employed to correct for target motion during acquisition phase. Registration is robust as to handle translational as well as rotational motion components. This ensures proper spectral composition. It is advantageous to be able to discern useful spectral data from extraneous data in the field of view. Methods for classifying useful data from all other data have been developed based on both spatial as well as spectral features. Such methods allow for higher accuracy and increased performance by eliminating from acquired dataset extraneous objects like hair, non-skin material (bandages, clothing, etc.), or any other objects that do not carry useful information about tissue hemoglobin content.

The system may extract tissue oxygenation values for oxyhemoglobin, deoxyhemoglobin and oxygen saturation, using the data acquired from a set of predetermined wavelengths via one or more spectral classification methods. Apart from making classification decisions based only on acquired data, a priori knowledge integration will take place effectively fusing an ensemble of classifiers to boost resulting clinical efficacy. Such data is extracted from clinical studies engaging diverse patient populations. Extracting robust "historical" (information across multiple visits) patient information and effectively presenting it for doctor's use can be achieved using well-established statistical techniques such as PCA (Principal Components Analysis), ICA (Independent Components Analysis) and LDA (partial least square) to isolate the most informative features therefore to improve the robustness and measurement accuracy of the system.

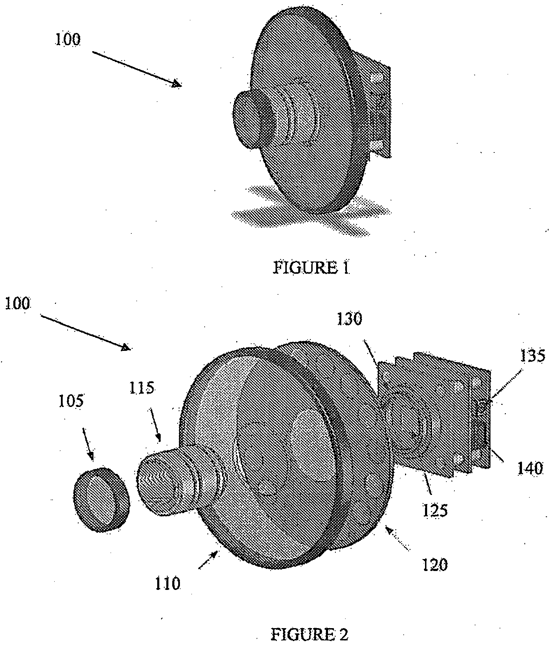

Figure 1 is a 3-D view of the multi-spectra imaging device 100. Preferably, device 100 is a handheld device having a maximum diameter of less than 50,8 cm (20 inches). More preferably the maximum diameter is less than 25,4 cm (10 inches)and even more preferably the diameter is less than 12,7 cm (5 inches). Device 100 is preferably self contained, and can be mounted on a tripod or arm extending from the wall of a hospital or clinical setting. Device 100 may be in wired or wireless communication with a user. Device 100 may have an internal or external power source.

Figure 2 is an exploded view of device 100. The device may include a lens polarizer assembly 105, a lens 110, an illumination polarizer assembly 115, an illumination module 120, an image sensor 125, and a lens mount 130. Device 100 may optionally include a power input 135 and/or a wired communications interface 140.

The handheld multi-spectral imaging system, is designed for tissue optical imaging for non-contact evaluation of tissue oxygenation, over an extended area, without harmful radiation, and without the need of using any agent into the tissue. The compact design with fast image sensor, high efficient illuminator and high-speed wavelength selection allows the device to be entirely portable and self-contained to acquire image data and provide hyperspectral information of tissue oxygenation to the user. The device may employ fast image acquisition and data processing with on-line processing using a combination of parallel processing circuits achievable with Field-Programmable Gate Arrays (FPGAs). The device may be capable of on-line display of the hyperspectral image on the handheld device via simple and intuitive graphic user interface for rapid view and sending image data to a remote computer for further processing and manipulation. Custom electronics and software allow for fast signal/imaging processing, analysis and display, and sending image data through wired and/or wireless connection for storage and may extract tissue oxygenation values for oxyhemoglobin, deoxyhemoglobin and oxygen saturation, using the data from a set of predetermined wavelengths use one or more tissue classification methods.

Several tissue masks may be applied to improve system performance by just focusing on the tissue of interest. Some tissue masks are spectral based and others are spatial based.

Since the device uses noncontact measurements, hemoglobin oxygenation status is not affected by how much pressures is placed on skin as with NIR probes. Measurement can also be made at a reasonably remote distance and through optical face shields if necessary. There is no need to disinfect system between patients as would be required for NIR systems.

The multi-spectral system uses visible wavelengths rather than NIR wavelengths which are more effectively absorbed by hemoglobin. In addition, because the photon pathlength is more superficial (~2mm), the multi-spectral imager predominantly measures hemoglobin in skin capillaries. As a result skin and fat layer thicknesses have less influence on the optical signals.

The multi-spectral imaging system captures hemoglobin oxygen saturation measurements over a reasonably wide field of view enabling the spatial variation to be measured. For example, subclinical skin mottling prior to the onset of shock, diabetic foot ulcers, claudication or other disease states can be measured with the spectral imager.

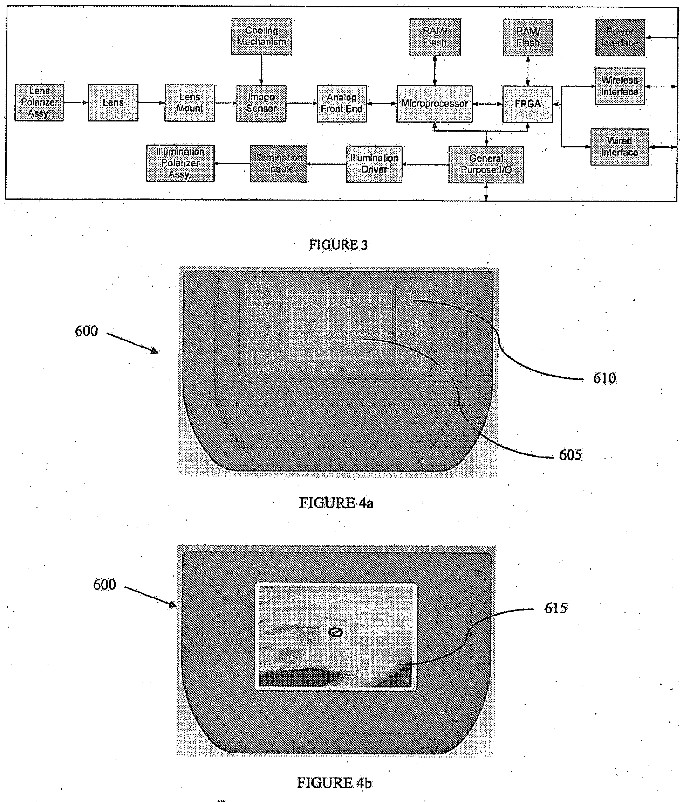

Figure 3 is a schematic block diagram of a multi-spectral imager. The image sensor is used for gathering multi-spectral data. While the Analog Front End (AFE) is a chipset that handles all functions related to conversion of analog signal data from image sensor to digital images. The digital image is then sent to the microprocessor, which is used to control all AFE parameters (gain, DC offset, brightness, exposure, etc.), temporarily store one or more images (RAM/Flash), interface with other modules via GPIO (illumination module), and interface with FGPA for image acquisition control and image dumping.

The Field Programmable Gate Array (FPGA) is used to control high level image acquisition and frame transfer (from RAM/Flash), interface with other modules via GPIO, processing of hypercube data, algorithm implementation, and interface with integrated wired and wireless communications modules. The illumination module is used to emit light of specified wavelengths during the image acquisition cycle.

Images collected at each wavelength may be spatially filtered to improve signal to noise ratios. The images may also be shifted accordingly so the pixels in each image represent the same site of the object plane. Spectral and spatial algorithms may be used to mask anything in the object plane that does not resemble tissue (e.g. the patients' clothing, hat or any other accessory, hair, dirt or grim, etc.). Oxyhemoglobin, deoxyhemoglobin and oxygen saturation may be extracted from data at each pixel identified as representing tissue using standard hemoglobin decomposition algorithms.

Figure 4a is a front view of another embodiment of the multi-spectral imager 600, while Figure 4b is a rear view of the embodiment. The multi-spectral imager 600 may be fitted with a disposable illumination cartridge. Multi-spectral imager 600 has a circuit board including at least one image sensor 605. Preferably there are between two and twenty image sensors. Sensors 605 may sense any wavelength including visible, color, near infrared, far infrared, or any combination thereof. Imager 600 may also include a lens for each image sensor 605. Between each lens and each image sensor 605 may be a filter. The filters may be set to filter out specific wavelengths. The filters may be optimized to produce the best detection. Additionally imager 600 may include an illumination source 610. The image sensors 605 and the illumination source 610 may all be located on the same circuit board. The circuit board may further include at least one Field-Programmable Gate Array (FPGA).

Imager 600 may include a display 615 to display a captured image. Display 615 may be a touch screen so that information can be entered through display 615 into imager 600. Display 615 may be of any size. Imager 600 may interface with an analysis device via an Ethernet connection, USB connection or wirelessly. Analysis device may be used for image to image comparisons, storage, and review of images. Imager 600 may be made of any material, including but not limited to, plastic and metal.

**Claims**

1.  A portable multi-spectral imaging device (100), comprising:

    at least one image acquisition device configured to acquire a multi-spectral image from a subject;
    a filtering device to filter light received by the image acquisition device;
    an acquisition subsystem configured to:

    continuously monitor quality of acquired image data with respect to the signal-to-noise ratio achieved, and perform motion tracking in real-time such that acquired data is not corrupted during imaging;

    an analogue front end module to convert the image to a digital image;
    a microprocessor to temporarily store at least one image and control the analog front end module; and
    a communication module configured to send image data through a wireless connection for storage.

2.  The device of claim 1, wherein the acquired image is used to determine oxygenation values of the subject.

3.  The device of claim 2, wherein the oxygenation values are based on at least one of oxyhemoglobin, deoxyhemoglobin and oxygen saturation levels.

4.  The device of claim 2, wherein the oxygenation values are based on component analysis.

5.  The device of claim 1, further comprising an illumination source (610), optionally, wherein the illumination source produces filtered light.

6.  The device of claim 5, further comprising an imaging acquisition parameter dynamics module configured to perform on-the-fly adjustment of image sensor parameters such that signal-to-noise requirements are met for a given target during image acquisition, including:

    (i) choosing appropriate wavelengths of the light produced by the illumination source;
    (ii) controlling high-speed wavelength selection of the light produced by the illumination source; and
    (iii) controlling high-efficiency illumination in real-time based on surface reflectance characteristics used as feedback at a given wavelength of light.

7.  The device of claim 1, wherein the microprocessor is further configured to execute instructions for adaptive spatial filtering to ensure that noise is minimized without compromising informative data.

8.  The device of claim 1, wherein the device has a maximum diameter of 50,8 cm (20 inches).

9.  The device of claim 1, wherein the device is handheld.

10. The device of claim 1, further including a power source.

11. The device of claim 1, wherein the at least one image acquisition device acquires images from wavelengths in the range of at least one of visible, color, near infrared, and far infrared.

**Patentansprüche**

1.  Ein tragbares Multispektral-Bildaufnahmegerät (100), umfassend:

mindestens ein Bilderfassungs-Gerät, eingerichtet, um ein Multispektral-Bild von einem Subjekt zu erfassen;
ein Filter-Gerät, um vom Bilderfassungsgerät empfangenes Licht zu filtern;
ein Erfassungs-Untersystem, eingerichtet um:

durchgehend die Qualität von erfassten Bilddaten im Bezug auf das erreichte Signal-Rausch-Verhältnis zu überwachen und
Bewegungsverfolgung in Echtzeit durchzuführen, sodass die erfassten Daten während der Aufnahme nicht fehlerhaft werden;

ein analoges Frontend-Modul, um das Bild in ein digitales Bild umzuwandeln;
einen Mikroprozessor, um mindestens ein Bild temporär zu speichern und das analoge Frontend-Modul zu kontrollieren; und
ein Kommunikations-Modul, eingerichtet, um Bilddaten zum Speichern über eine drahtlose Verbindung zu senden.

2. Das Gerät von Anspruch 1, wobei das erfasste Bild verwendet wird, um Oxygenierungswerte des Subjekts zu bestimmen.

3. Das Gerät von Anspruch 2, wobei die Oxygenierungswerte auf mindestens einem von Oxyhämoglobin, Desoxyhämoglobin und Sauerstoffsättigungsspiegeln basieren.

4. Das Gerät von Anspruch 2, wobei die Oxygenierungswerte auf Komponentenanalyse basieren.

5. Das Gerät von Anspruch 1, außerdem umfassend eine Beleuchtungsquelle (610), wobei die Beleuchtungsquelle optional gefiltertes Licht produziert.

6. Das Gerät von Anspruch 5, außerdem umfassend ein Bilderfassungs-Parameterdynamik-Modul, eingerichtet, um spontan Anpassungen von Bildsensor-Parametern durchzuführen, sodass Signal-Rausch-Erfordernisse für ein gegebenes Ziel während der Bilderfassung erfüllt werden, umfassend

(i) Auswählen geeigneter Wellenlängen des von der Beleuchtungsquelle produzierten Lichts;
(ii) Kontrollieren der Hochgeschwindigkeits-Wellenlängenauswahl des von der Beleuchtungsquelle produzierten Lichts; und
(iii) Kontrollieren der Hocheffizienz-Beleuchtung in Echtzeit, basiert auf Oberflächenreflektivitäts-Charakteristika, die bei einer gegebenen Lichtwellenlänge als Rückmeldung verwendet werden.

7. Das Gerät von Anspruch 1, wobei der Mikroprozessor außerdem eingerichtet ist, Anweisungen für adaptives räumliches Filtern auszuführen, um sicherzustellen, dass Rauschen minimiert wird, ohne die Informationsdaten zu beeinträchtigen.

8. Das Gerät von Anspruch 1, wobei das Gerät einen maximalen Durchmesser von 50,8 cm (20 Zoll) hat.

9. Das Gerät von Anspruch 1, wobei das Gerät ein Handgerät ist.

10. Das Gerät von Anspruch 1, außerdem umfassend eine Energiequelle.

11. Das Gerät von Anspruch 1, wobei das mindestens eine Bilderfassungsgerät Bilddaten von Wellenlängen im Bereich von mindestens einem von sichtbar, Farbe, nahes Infrarot und fernes Infrarot erfasst.

**Revendications**

1. Dispositif portatif d'imagerie multispectrale (100), comprenant :

au moins un dispositif d'acquisition d'image configuré pour acquérir une image multispectrale depuis un sujet ;
un dispositif filtrant pour filtrer une lumière reçue par le dispositif d'acquisition d'image ;
un sous-système d'acquisition configuré pour :

surveiller en continu la qualité de données d'image acquises concernant le rapport signal sur bruit atteint, et effectuer une capture de mouvement en temps réel de sorte que les données acquises ne soient pas corrompues pendant la capture d'image ;

un module frontal analogique pour convertir l'image en une image numérique ;
un microprocesseur pour stocker provisoirement au moins une image et commander le module frontal analogique ; et
un module de communication configuré pour envoyer des données d'image par l'intermédiaire d'une connexion sans fil en vue du stockage.

2. Dispositif selon la revendication 1, dans lequel l'image acquise est utilisée pour déterminer des valeurs d'oxygénation du sujet.

3. Dispositif selon la revendication 2, dans lequel les valeurs d'oxygénation sont basées sur au moins l'un parmi des niveaux d'oxyhémoglobine, de désoxyhémoglobine et de saturation en oxygène.

4. Dispositif selon la revendication 2, dans lequel les valeurs d'oxygénation sont basées sur une analyse de composantes.

5. Dispositif selon la revendication 1, comprenant en outre une source d'éclairage (610), optionnellement, dans lequel la source d'éclairage produit une lumière filtrée.

6. Dispositif selon la revendication 5, comprenant en outre un module de dynamique de paramètres d'acquisition d'image configuré pour effectuer un réglage à la volée de paramètres de capteur d'image de sorte que des exigences de signal sur bruit soient satisfaites pour une cible donnée pendant une acquisition d'image, comportant :

(i) le choix de longueurs d'ondes appropriées de la lumière produite par la source d'éclairage ;
(ii) la commande d'une sélection de longueur d'onde à grande vitesse de la lumière produite par la source d'éclairage ; et
(iii) la commande d'un éclairage de haute efficacité en temps réel sur la base de caractéristiques de réflectivité de surface utilisées en tant que retour à une longueur d'onde donnée de la lumière.

7. Dispositif selon la revendication 1, dans lequel le microprocesseur est en outre configuré pour exécuter des instructions pour un filtrage spatial adaptatif afin d'assurer la minimisation du bruit sans compromettre les données d'information.

8. Dispositif selon la revendication 1, dans lequel le dispositif présente un diamètre maximal de 50,8 cm (20 pouces).

9. Dispositif selon la revendication 1, dans lequel le dispositif est portable.

10. Dispositif selon la revendication 1, comportant en outre une source d'alimentation électrique.

11. Dispositif selon la revendication 1, dans lequel l'au moins un dispositif d'acquisition d'image acquiert des images à partir de longueurs d'onde dans la plage d'au moins l'un parmi le visible, le coloré, l'infrarouge proche et l'infrarouge lointain.

100

FIGURE 1

100

130

135

105

115

140

110

125

120

FIGURE 2

FIGURE 3

600

610

605

FIGURE 4a

600

615

FIGURE 4b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007024946 A
- US 2008188758 A
- US 6640130 B

**Non-patent literature cited in the description**

- **SOWA, M. G. et al.** *Proc. SPIE,* 1998, vol. 3252, 199-207
- **SOWA, G. W. et al.** *Journal of Surgical Research,* 1999, vol. 86, 62-29
- **SOW, G. W. et al.** *Journal of Biomedical Optics,* 1999, vol. 4, 474-481
- **MANSFIELD, J. R. et al.** *International Society of Optical Engineers,* 2000, vol. 3920, 99-197
- **DE-BLASI, R. A. et al.** *Adv. Exp. Med. Biol,* 1992, vol. 317, 771-777
- **STRANC, M. F. et al.** *British Journal of Plastic Surgery,* 1998, vol. 51, 210-218
- **MANSFIELD, J. R. et al.** *Analytical Chemistry,* 1997, vol. 69, 3370-3374
- **MANSFIELD, J. R. et al.** *Computerized Medical Imaging and Graphics,* 1997, vol. 21, 299-308
- **SALZER, R. et al.** *Fresenius Journal of Analytical Chemistry,* 2000, vol. 366, 712-726
- **SHAW, R. A. et al.** *Journal of Molecular Structure (Theochem),* 2000, vol. 500, 129-138
- **SHAW, R. A. et al.** *Journal of Inorganic Biochemistry,* 2000, vol. 79, 285-293
- **MANSFIELD, J. R. et al.** *Proc. SPIE Int. Soc. Opt. Eng.,* 1999, vol. 3597, 222-233
- **MANSFIELD, J. R. et al.** *Applied Spectroscopy,* 1999, vol. 53, 1323-1330
- **MCINTOSH, L. M. et al.** *Biospectroscopy,* 1999, vol. 5, 265-275
- **MANSFIELD, R. et al.** *Vibrational Spectroscopy,* vol. 19, 33-45
- **PAYETTE, J. R. et al.** *Laboratory,* 1999, vol. 18, 4-6
- **MANSFIELD, J. R.** *IEEE Transactions on Medical Imaging,* 1998, vol. 6, 1011-1018
- **SOWA, M. G. et al.** *Applied Spectroscopy,* 1997, vol. 51, 143-152
- **LEVENTON, M.** *MIT Ph.D. Thesis,* 2000
- **HORN, B. K. P.** *Journal of the Optical Society of America A,* 1987, vol. 4, 629-642
- **MANDAVA, V. R. et al.** *Proc SPIE,* 1992, vol. 1652, 271-282
- **HARALICK, R. M. et al.** *Computer and Robot Vision,* 1993
- **WEST, J. et al.** *Proc SPIE, Newport Beach, Calif,* 1996
- **MAINTZ, J. B. ANTIONE et al.** *Computer Vision, Virtual Reality and Robotics in Medicine,* 1995, 219-228
- **MAGUIRE, JR., G. et al.** IEEE Computer Graphics Applications. 1991, vol. 11, 20-29
- **JIANG, H. et al.** *Proc. SPIE,* 1992, vol. 1808, 196-213
- **LEMOINE, D. et al.** *Proc. SPIE,* 1994, vol. 2164, 46-56
- **PELIZZARI, C. et al.** *J Comput Assist. Tumour.,* 1989, vol. 13, 20-26
- **ETTINGER, G.** *MIT Ph.D Thesis,* 1997
- **VAN DEN ELSEN, P. et al.** Visualization in Biomedical Computing. *Proc SPIE,* 1994, vol. 2359, 227-237
- **BELL, A. J. et al.** *Advances in Neural Information Processing,* 1995, vol. 7
- **COLLIGNON, D. et al.** *First Conf. on Computer Vision, Virtual Reality and Robotics in Medicine Springer,* 1995
- **MAES, F. et al.** *Mathematical Methods in Biomedical Image Analysis,* 1996
- **WELLS, W. M. et al.** *Medical Image Analysis,* 1996, vol. 1 (1), 35-51
- **LEVENTON, M. et al.** *Medical Image Computing and Computer-assisted Intervention,* 1998